(19) Europäisches Patentamt
European Patent Office
Office européen des brevets

(11) Publication number: **0 563 112 B1**

(12) **EUROPEAN PATENT SPECIFICATION**

(45) Date of publication of patent specification: **18.01.95**

(51) Int. Cl.[6]: **A61K 9/08**, A61K 9/48, A61K 9/66, A61K 47/14, A61K 31/10

(21) Application number: **92900932.2**

(22) Date of filing: **15.11.91**

(86) International application number: **PCT/US91/08565**

(87) International publication number: **WO 92/10996 (09.07.92 92/17)**

# (54) ENHANCED BIOAVAILABILITY PHARMACEUTICAL COMPOSITION CONTAINING PROBUCOL.

(30) Priority: **18.12.90 US 629540**

(43) Date of publication of application: **06.10.93 Bulletin 93/40**

(45) Publication of the grant of the patent: **18.01.95 Bulletin 95/03**

(84) Designated Contracting States: **AT BE CH DE DK ES FR GB GR IT LI LU NL SE**

(56) References cited:
**US-A- 3 862 332**
**US-A- 4 902 513**

(73) Proprietor: **MERRELL DOW PHARMACEUTICALS INC.**
**P.O. Box 156300**
**2110 East Galbraith Road**
**Cincinnati**
**Ohio 45215-6300 (US)**

(72) Inventor: **BASSLER, Kenneth G.**
**303 John Street**
**Carmel, IN 46032 (US)**
Inventor: **DEEPAK, Phadke S.**
**8827 Ginnylock Drive**
**Indianapolis, IN 46256 (US)**
Inventor: **NEDDERMEYER, Melissa P.**
**5427 Ginnylock Drive**
**Indianapolis, IN 46255 (US)**

(74) Representative: **Barth, Renate, Dr.**
**Vossius & Partner**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

Probucol, a serum cholesterol lowering agent, is presently marketed as Lorelco® 250 and 500 mg tablets. The bioavailability of probucol from tablet dosage form is estimated to be 2-8 percent [J.F. Heeg and H. Tachizawa, Nouv. Presse Med., 9, 2990-2994 (1980)]. This poor bioavailabllity is most likely caused by the extremely hydrophobic nature of probucol. Several approaches for improving the bioavailability of poorly water soluble drugs have been reported in the literature. Drugs are absorbed from the gastrointestinal tract most rapidly when administered as aqueous solutions. The absorption rate of a drug from an oil solution may be enhanced, however, if the oil is digestible. Therefore, it was considered appropriate to develop an oil solution formulation of probucol filled in a hard gelatin capsule as one of the approaches to improving its bioavailability. In developing such a formulation, it was unexpectedly discovered that one such formulation that increased bioavailability also resulted in reduced variability of plasma drug levels of probucol in a patient population to which the formulation was administered.

Three new pharmaceutical dosage forms of probucol were prepared and the relative bioavailability was evaluated in man. One of these dosage forms, a solution of probucol in Captex® 200 filled in hard gelatin capsules, was found (by extrapolation) to be approximately equal to the Lorelco® 500 tablet at about 1/6 the dose. The solubility of probucol was determined in several natural and derived vegetable oils. Captex® 200, a vegetable oil containing propylene glycol esters of caprylic ($C_8$) and capric ($C_{10}$) fatty acids, provided the highest solubility for probucol and was therefore selected as the preferred vehicle for an improved probucol formulation. Also, unexpectedly, there was significantly less variability in probucol plasma drug levels with the Captex® 200 formulation. In view of the increased bioavailability of probucol when administered in the Captex® 200 formulation and in the light of the unexpected reduced variability in probucol plasma levels, this formulation is the subject of this application.

Probucol is a compound according to Formula I, namely 2,2'-bis (3,5-di-tertiarybutyl-4-hydroxyphenylthio)propane.

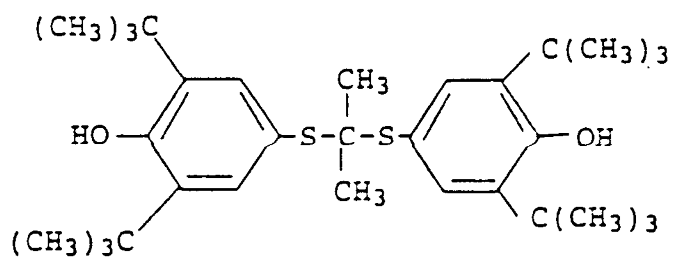

FORMULA I

The compounds of Formula I can be prepared as described in U.S. Patents Nos. 3,576,883, 3,786,100, 3,862,332, 3,987,500 and 4,900,757, incorporated herein by reference. More specifically, 2,2'-bis (3,5-di-tertiarybutyl-4-hydroxy phenylthio)propane can be prepared as described in U.S. Patent No. 3,576,883, also incorporated herein by reference. Alternatively, this compound can be prepared according to the method set forth in U.S. Patent Nos. 4,734,527 (Kraus) or 4,861,443 (Van Effen), incorporated herein by reference. The indication for probucol is primary hypercholesterolemia. Recent studies in animals have indicated that probucol has a beneficial effect on atherosclerosis independent of cholesterol lowering.

The present invention is directed towards pharmaceutical compositions of probucol dissolved in a propylene glycol ester of fatty acids wherein the fatty acids are selected from the group consisting of the fatty acids represented by $C_xH_{2x}O_2$, wherein x is 4, 6, 8, 10, 12, 14, 16. This group specifically includes butyric acid, caproic acid, caprylic acid, capric acid, lauric acid, myristic acid and palmitic acid. The most preferred embodiment of the invention is a propylene glycol ester of capric and caprylic acids, known as propylene glycol dicaprylate/dicaprate. Captex® 200 is a specific trade name for propylene glycol dicaprylate/dicaprate and is supplied by Karlshamns Lipid Specialties USA, P.O. Box 569, Columbus, OH 43216-0569. Reference to Captex® 200 should not be construed as limiting and it will be understood that any reference to Captex® 200 should be construed to generically include all propylene glycol dicaprylates/dicaprates. Propylene glycol dicaprylate/dicaprate is also known as Neobee 20, supplied by Stepan Co., PVO Dept., 100 W. Hunter Ave., Maywood, NJ 07607, and as Miglyol 840 , supplied by Huls America, P.O. Box 456, Piscataway, NJ 08855-0456. Captex® 300 and Capmul MCM is also supplied by Karlshamns Lipid Specialties USA.

SOLUBILITY DETERMINATION

The solubility of probucol was determined in olive, sunflower, peanut, vegetable, corn, Captex® 200 and 300, and Capmul® MCM oils. Captex® 200 is a propylene glycol ester of caprylic ($C_8$) and capric ($C_{10}$) fatty acids obtained by fractionation of certain coconut oil fatty acids and is known generically as propylene glycol dicaprylate/dicaprate. Captex® 300 is a caprylic and capric acid triglyceride obtained by fractionation and subsequent esterification of coconut oil and is known generically as caprylic/capric triglyceride. Capmul® MCM is a mono and diglyceride of caprylic and capric acids.

Eight grams of each oil were transferred into a glass tube with a teflon liner screw cap, and 2.5 g of probucol were added to each tube. The tubes were capped and shaken by hand until the probucol particles were wetted. The tubes were then rotated for at least 48 hours on a test tube rotating apparatus. The solubility of probucol was determined using a high performance liquid chromatography (HPLC) assay procedure.

The solubility values (%w/v) of probucol in various oils are shown in Table I.

TABLE I

| SOLUBILITY OF PROBUCOL IN VARIOUS OILS | | | |
|---|---|---|---|
| Oil | Solubility (% w/v) | Oil | Solubility (% w/v) |
| Peanut | 5.6 | Corn | 5.8 |
| Olive | 5.5 | Captex® 200 | 18.2 |
| Sunflower | 5.8 | Captex® 300 | 12.5 |
| Safflower | 5.9 | Capmul® MCM | 6.3 |
| Vegetable | 5.8 | | |

The highest solubility was observed in Captex® 200 oil. Considering a 50 mg probucol dose, and the constraints on the capsule size and its fill volume, the Captex® 200 was selected for further study. Although coconut oil is known to increase the serum cholesterol level, the literature on Captex® oil indicated that the medium chain fatty acids present in this oil do not contribute to the increase in the cholesterol level. Additionally, there is also evidence that these acids are absorbed through the portal system [V.K. Babayan, Lipids, 22, 417-420 (1987)] and may actually lower the cholesterol level [J.W. Stewart, K.D. Wiggers, N.L. Jacobson, P.J. Berger, Journal of Nutrition, 108, 561-566 (1978) and D. Kritchevsky, S.A. Tepper, Journal of Nutrition, 86, 67-72 (1965)]. Although the exact mechanism of action of probucol is not completely understood, there is speculation that its primary mechanism of action is in the liver. The portal absorption of the fatty acids present in Captex® 200 may be an advantage if probucol is to exercise its action mainly in the liver.

In order to determine if the solubility of probucol could be enhanced by incorporating absolute ethanol in the oil, three binary systems: safflower oil:ethanol (90:10), polyethylene glycol (PEG) 400:ethanol (90:10, 80:20, 70:30), and Captex® 200:ethanol (95:5, 90:10, 85:15, 80:20, and 75:25) were evaluated. The solubility data shown in Table II indicate that in each case the solubility of probucol increased in the presence of ethanol.

TABLE II

| SOLUBILITY OF PROBUCOL IN VARIOUS BINARY SOLVENT SYSTEMS | | |
|---|---|---|
| Solvent System | Ratio | Solubility (%w/v) |
| Safflower Oil:Ethanol | 90:10 | 11.5 |
| PEG 400 | 100:00 | 2.8 |
| PEG 400:Ethanol | 90:10 | 5.8 |
| PEG 400:Ethanol | 80:20 | 9.5 |
| PEG 400 Ethanol | 70:30 | 13.1 |
| Captex® 200:Ethanol | 95:5 | 23.0 |
| Captex® 200:Ethanol | 90:10 | 24.0 |
| Captex® 200:Ethanol | 85:15 | 25.0 |
| Captex® 200:Ethanol | 80:20 | 26.0 |
| Captex® 200:Ethanol | 75:25 | 26.0 |

BIOAVAILABILITY AND SERUM VARIABILITY STUDIES

Further studies were conducted to assess the bioavailability of the experimental formulations of probucol. Two studies were conducted as open, randomized, parallel studies with twelve subjects per treatment group. Lorelco® 500 mg tablets were used as the reference formulation and compared with a Scherer soft gelatin capsule (Protocol A) and Captex® Oil Solution and PEG (polyethylene glycol) 8000 comelt (Protocol B), respectively, each containing 50 mg of probucol.

The current formulation of Lorelco® is 500 mg of probucol in admixture with corn starch, ethylcellulose, glycerine, hydroxypropyl cellulose, hydroxypropyl methylcellulose 2910, iron oxide, lactose, magnesium stearate, microcrystalline cellulose, polysorbate 80, talc and titanium dioxide. PEG 8000 comelt is a mixture of probucol and polyethylene glycol 8000 (PEG 8000 is known in the art). Size two gelatin capsules were filled with 100 mg or the 50:50 probucol:PEG 8000 comelt, corresponding to a 50 mg dose of probucol. The Scherer soft gel is a mixture of fill weight, 310 mg consisting of 50 mg probucol, 208 mg Captex® 200, 26 mg polysorbate 80 and 26 mg Imwitor 742 (caprylic/capric glycerides-HULS America).

Summary statistics (mean, standard deviation, and coefficient of variation) for dose corrected pharmacokinetic parameters are listed in Tables III and IV for Protocols B and A respectively.

TABLE III

| PROTOCOL B - Summary Statistics For Dose Corrected Pharmacokinetic Parameters Mean ± S.D., (C.V.), (N = 12) | | | |
|---|---|---|---|
| Dose Corrected Parameters | Lorelco® | Captex® Oil | PEG 8000 |
| AUC168 ($\mu g{*}hr\ ml^{-1}$) | 162 ± 108 (67%) | 919 ± 127 (14%) | 979 ± 363 (37%) |
| $C_{MAX}$ ($\mu g/ml$) | 2.32 ± 1.50 (65%) | 13.5 ± 2.72 (20%) | 13.9 ± 4.23 (30%) |
| $T_{MAX}$ (hr) | 20.0 ± 7.24 (36%) | 18.3 ± 7.13 (39%) | 20.0 ± 7.43 (37%) |

TABLE IV

| PROTOCOL A - Summary Statistics For Dose Corrected Pharmacokinetic Parameters Mean ± S.D., (C.V.), (N = 12) | | |
|---|---|---|
| Dose Corrected Parameters | Lorelco® | Scherer soft gel |
| AUC168 ($\mu g_* hr\ ml^{-1}$) | 276 ± 126 (47%) | 1017 ± 328 (32%) |
| $C_{MAX}$ ($\mu$g/ml) | 3.74 ± 1.55 (41%) | 14.2 ± 3.49 (25%) |
| $T_{MAX}$ (hr) | 18.7 ± 6.89 (37%) | 19.5 ± 7.14 (37%) |

For Protocol B, based on the dose corrected mean AUC (area under the curve) and $C_{max}$ (maximum concentration) values, both Captex® Oil solution and PEG 8000 comelt are estimated to be 5 times or more bioavailable than the Lorelco® 500 mg tablet (Table III). $T_{max}$ values are similar for all three formulations. To make a fair comparison of the variabilities of the test formulations (Captex® Oil Solution and PEG comelt), and the reference formulation (Lorelco® 500 mg tablet), AUC and $C_{max}$ values for Captex® Oil Solution and PEG 8000 comelt were multiplied by 1.76 and 1.65, respectively (these scale factors were used so that all formulations have the same AUC values). Based on the standard deviation of these extrapolated values (Table V) and the coefficient of variation of the raw values (Table III), AUC values of Captex® Oil Solution are approximately 25 and 7 times less variable than the Lorelco® tablet and the PEG 8000 treatment, respectively, and $C_{max}$ values of Captex® Oil Solution are approximately 9 and 2.3 times less variable than the Lorelco® tablet and PEG 8000 treatment, respectively. The variability of PEG 8000 comelt is similar to the Lorelco® 500 mg tablet.

Similar procedures were also used for Protocol A. Based on the dose corrected values, the Scherer soft gel treatment is estimated to be 3.7 times more bioavailable than the Lorelco® 500 mg tablet (Table IV). $T_{max}$ values are similar for both formulations. The variability of Scherer soft gel treatment is similar to the Lorelco® 500 mg tablet (Table V).

TABLE V

Comparison Of Variances With Matched AUC Values

Standard Deviation Of The Extrapolated Values (N=12)

PROTOCOL B

| Parameter | Lorelco® (Treatment*) | PEG 8000 (Treatment*) | Captex® Oil (Treatment*) |
|---|---|---|---|
| AUC168 (μg*hr ml-1) | 108.2 | 61.8 | 22.9 |
| CMAX (μg/ml) | 1.50 | 0.72 | 0.49 |
| TMAX (hr) | 7.20 | 7.10 | 7.40 |

PROTOCOL A

| Parameter | Lorelco® (Treatment*) | Scherer soft gel (Treatment*) |
|---|---|---|
| AUC168 (μg*hr ml-1) | 125.7 | 86.9 |
| CMAX (μg/ml) | 1.55 | 0.94 |
| TMAX (hr) | 6.90 | 7.10 |

*Treatments with a common bracket are not significantly different.

EXAMPLE I

Probucol (50.0 mg) was dissolved in propylene glycol esters of caprylic/capric fatty acids [Captex® 200, manufactured and supplied by Karlshamns-Lipid Specialties USA, P.O. Box 569, Columbus, OH 43216-0569, as Captex® 200] (283.0 mg) and stirred until a clear solution was obtained. The resulting clear solution was filled into hard gelatin capsules (white opaque gelatin capsule size no. 1, 73 mg) so that each capsule contained an approximate weight of 333.0 mg of solution. The bulk solution was assayed for probucol before filling the capsule and the fill weight was adjusted according to the actual percent of probucol in the solution to provide a 50 mg dose of probucol. Using a capsule banding apparatus, a solution of gelatin (0.647 mg), polysorbate 80 (0.027 mg), and purified water (2.076 mg) was applied to seal the cap to the body of the capsule. The gelatin band was then allowed to harden.

**Claims**

1. A pharmaceutical composition of probucol adapted to enhance the bioavailability of probucol while reducing plasma probucol level variability in a patient population comprising a therapeutically effective amount of probucol dissolved in a propylene glycol ester of fatty acids wherein the fatty acids are selected from the group consisting of the fatty acids represented by $C_xH_{2x}O_2$, wherein x is 4, 6, 8, 10, 12, 14, 16.

2. A pharmaceutical composition according to claim 1 wherein the fatty acids are selected from the group consisting of the fatty acids represented by $C_xH_{2x}O_2$, wherein x is 6, 8, 10, 12.

3. A pharmaceutical composition according to claim 1 wherein the fatty acids are selected from the group consisting of capric and caprylic acids.

4. Use of probucol dissolved in a propylene glycol ester of fatty acids wherein the fatty acids are selected from the group consisting of the fatty acids represented by $C_xH_{2x}O_2$, wherein x is 4, 6, 8, 10, 12, 14, 16 for the preparation of a pharmaceutical composition to lower serum cholesterol levels.

5. A method for the preparation of a pharmaceutical composition of probucol adapted to enhance the bioavailability of probucol while reducing plasma probucol level variability in a patient population comprising dissolving a therapeutically effective amount of probucol in a propylene glycol ester of fatty acids wherein the fatty acids are selected from the group consisting of the fatty acids represented by $C_xH_{2x}O_2$, wherein x is 4, 6, 8, 10, 12, 14, 16.

6. A method according to claim 5 wherein the fatty acids are selected from the group consisting of the fatty acids represented by $C_xH_{2x}O_2$, wherein x is 6, 8, 10, 12.

7. A method according to claim 5 or 6 wherein the fatty acids are selected from the group consisting of capric and caprylic acids.

**Patentansprüche**

1. Probucol-haltiges Arzneimittel, das die Bioverfügbarkeit von Probucol erhöht, während es Veränderungen des Plasmaprobucolspiegels von Patienten verringert, umfassend eine therapeutisch wirksame Menge von Probucol, das in mit Propylenglykol veresterten Fettsäuren gelöst ist, wobei die Fettsäuren aus Fettsäuren der Formel $C_xH_{2x}O_2$ ausgewählt sind, in der x den Wert 4, 6, 8, 10, 12, 14 oder 16 hat.

2. Arzneimittel nach Anspruch 1, wobei die Fettsäuren aus Fettsäuren der Formel $C_xH_{2x}O_2$ ausgewählt sind, in der x den Wert 6, 8, 10 oder 12 hat.

3. Arzneimittel nach Anspruch 1, wobei die Fettsäuren aus Caprin- und Caprylsäuren ausgewählt sind.

4. Verwendung von Probucol, das in mit Propylenglykol veresterten Fettsäuren gelöst ist, wobei die Fettsäuren aus Fettsäuren der Formel $C_xH_{2x}O_2$ ausgewählt sind, in der x den Wert 4, 6, 8, 10, 12, 14 oder 16 hat, zur Herstellung eines Arzneimittels zur Verringerung der Serumcholesterinspiegel.

5. Verfahren zur Herstellung eines Probucol-haltigen Arzneimittels, das die Bioverfügbarkeit von Probucol erhöht, während es Veränderungen des Plasmaprobucolspiegels von Patienten verringert, umfassend das Auflösen einer therapeutisch wirksamen Menge von Probucol in mit Propylenglykol veresterten Fettsäuren, wobei die Fettsäuren aus Fettsäuren der Formel $C_xH_{2x}O_2$ ausgewählt sind, in der x den Wert 4, 6, 8, 10, 12, 14 oder 16 hat.

6. Verfahren nach Anspruch 5, wobei die Fettsäuren aus Fettsäuren der Formel $C_xH_{2x}O_2$ ausgewählt sind, in der x den Wert 6, 8, 10 oder 12 hat.

7. Verfahren nach Anspruch 5 oder 6, wobei die Fettsäuren aus Caprin- und Caprylsäuren ausgewählt sind.

**Revendications**

1. Composition pharmaceutique de probucol adaptée pour améliorer la biodisponibilité du probucol tout en réduisant la variabilité du taux de probucol plasmatique dans une population de patients, comprenant une quantité efficace du point de vue thérapeutique de probucol dissoute dans un ester de propylène glycol d'acides gras dans lequel les acides gras sont choisis dans le groupe constitué d'acides gras représentés par $C_xH_{2x}O_2$, dans lequel x est 4, 6, 8, 10, 12,14, 16.

2. Composition pharmaceutique selon la revendication 1, dans laquelle les acides gras sont choisis dans le groupe constitué d'acides gras représentés par $C_xH_{2x}O_2$, dans lequel x est 6, 8, 10, 12.

3. Composition pharmaceutique selon la revendication 1, dans laquelle les acides gras sont choisis dans le groupe constitué d'acides caprique et caprylique.

4. Utilisation de probucol dissous dans un ester de propylène glycol d'acides gras dans lequel les acides gras sont choisis dans le groupe constitué d'acides gras représentés par $C_xH_{2x}O_2$, dans lequel x est 4, 6, 8, 10, 12, 14, 16 pour la préparation d'une composition pharmaceutique destinée à abaisser les taux de cholestérol sérique.

5. Procédé pour la préparation d'une composition pharmaceutique de probucol adaptée pour améliorer la biodisponibilité tout en réduisant la variabilité du taux de probucol plasmatique dans une population de patients, comprenant la dissolution d'une quantité efficace du point de vue thérapeutique de probucol dans un ester de propylène glycol d'acides gras dans lequel les acides gras sont choisis dans le groupe constitué d'acides gras représentés par $C_xH_{2x}O_2$, dans lequel x est 4, 6, 8, 10, 12, 14, 16.

6. Procédé selon la revendication 5, dans laquelle les acides gras sont choisis dans le groupe constitué d'acides gras représentés par $C_xH_{2x}O_2$, dans lequel x est 6, 8, 10, 12.

7. Procédé selon la revendication 5 ou 6, dans laquelle les acides gras sont choisis dans le groupe constitué d'acides caprique et caprylique.